# EUROPEAN PATENT APPLICATION

(11) **EP 3 248 570 A1**
(43) Date of publication of application: **29.11.2017**
(21) Application number: 16740103.3
(22) Date of filing: 18.01.2016
(51) Int. Cl.: A61C 19/00, A61H 39/04

(54) **ORAL MOUNTING FIXTURE**

(30) Priority: 20.01.2015 JP 2015008243; 21.08.2015 JP 2015163433
(71) Applicant: Satake, Shuichi, Chikusei-city, Ibaraki 300-4525 (JP)
(72) Inventor: Satake, Shuichi, Chikusei-city, Ibaraki 300-4525 (JP)
(74) Representative: Dennemeyer & Associates S.A.
(86) International application number: PCT/JP2016/051263
(87) International publication number: WO 2016/117496

(57) **Abstract**

The obtained oral mounting fixture can be used in public such as during conversation with other people when halitosis is of particular concern and can be used at any time with no limit to the timing of use, and thus further effectively promotes the secretion of saliva. The oral mounting fixture configured to be mounted inside an oral cavity of a human and promote the secretion of saliva has a main body section 1 that can be detachably mounted on a part in the oral cavity, and having a shape arranged so that the main body section 1 conforms to the shape of a portion on which the oral mounting fixture is mounted, and plural protrusions 2 that are integrally formed in the main body section 1 and protruding from the surface of the main body section 1. This oral mounting fixture is mounted so that the tips of the plural protrusions 2 press on a mucosa in the oral cavity.

## Description

### TECHNICAL FIELD

The present invention relates to an oral mounting fixture. The present invention specifically relates to an oral mounting fixture that is mounted in an oral cavity and effectively promotes the secretion of saliva to thereby prevent halitosis.

### BACKGROUND ART

Many people worry about halitosis during conversation with other people. Generally, countermeasures for preventing halitosis such as thorough brushing of teeth after meal, washing liquids for preventing halitosis, sprays for preventing halitosis and supplements for preventing halitosis are used.

Furthermore, an apparatus for administering a deodorant agent and an air freshener and the like for preventing halitosis by diffusion in a continuous manner with natural feeling without causing unpleasant sensation and repulsion is described in, for example, Patent Literature 1.

Furthermore, Patent Literature 2 describes that a mouth piece is pressed against the insides of the both buccas in an oral cavity and the both buccas are put into a state that they are pushed upward to stimulate the entirety in the oral cavity to thereby allow secretion of a large amount of saliva.

Furthermore, Patent Literature 3 describes the following points. An instrument to promote the secretion of saliva is inserted in between a maxillary gum part in an oral cavity and an upper lip. A chewing part is inserted to and withdrawn from between upper teeth and lower teeth by using a tongue, and then slightly chewed. By this way, the secretion of saliva is promoted, halitosis is suppressed, and conversation is enabled without any problem.

Furthermore, more saliva than usual is secreted by the chewing. Therefore, Patent Literature 4 describes a chewing exercise tool to be mounted along a lower dentition.

### CITATION LIST

### PATENT LITERATURE

Patent Literature 1: JP 2001-258910 A
Patent Literature 2: JP 3167198 U
Patent Literature 3: JP 2011-417 A
Patent Literature 4: JP 2001-128994 A

### SUMMARY OF INVENTION

### PROBLEMS TO BE SOLVED BY THE INVENTION

The content of the description of Patent Literature 1 is not to promote the secretion of saliva by massaging ducts of the major salivary glands during mounting but to prevent halitosis by administering a drug. Therefore, it is difficult to effectively promote the secretion of saliva.

The content of the description of Patent Literature 2 is not that stimulation is strengthened and weakened as in massaging but that the both buccas are put into a state that they are pushed upward by mounting a flexible mouth piece in the oral cavity. This is insufficient to effectively promote the secretion of saliva as in the content of the description of Patent Literature 1.

The content of the description of Patent Literature 3 is to promote the secretion of saliva by chewing the chewing part, and thus is fundamentally different from stimulating salivary glands. In this method, the secretion of saliva cannot be effectively promoted during mounting. The content of the description of Patent Literature 4 is also that more saliva than usual is secreted by the chewing, and is not that promotion of the secretion of saliva during mounting.

The object of the present invention is to solve the problems of the above-mentioned conventional technologies, and to enable use in public such as during conversation with other people when halitosis is of particular concern, enable use at any time with no limit to the timing of use, and to further effectively promote the secretion of saliva.

### SOLUTIONS TO THE PROBLEMS

In order to the achieve above-mentioned object, the oral mounting fixture configured to be mounted inside an oral cavity of a human and promote the secretion of saliva of the present invention includes a main body section that can be detachably mounted on a part in the oral cavity, and having a shape arranged so that the main body section conforms to the shape of a portion on which the oral mounting fixture is mounted, and plural protrusions that are integrally formed in the main body section and protruding from the surface of the main body section, wherein the oral mounting fixture is mounted so that the tips of the plural protrusions press on a mucosa in the oral cavity.

Furthermore, it is desirable that the above-mentioned oral mounting fixture is mounted so that the tips of the plural protrusions press on at least either of an inner surface part being a buccal mucosa of the oral cavity, a submandibular part between a mandibular dentition and a lingual base and a sublingual part being a floor of the oral cavity between a dentition and a lingual base.

Furthermore, in the above, it is preferable that the protrusions each has a rod-like shape and are formed in the form of bristles from the main body section.

Furthermore, it is preferable that the oral mounting fixture has a fitting part that can be fitted to the dentition.

Furthermore, in the above, it is desirable that the main body section has plural fitting parts capable of fitting to the dentition, and the fitting parts are linked by a string-like linking part.

Furthermore, in the above, it is desirable that the protrusions have plural buccal protrusions configured to press on an inner surface part of an oral cavity and plural gingiva protrusions configured to press on a gingiva, wherein the gingiva protrusions each has a smaller protrusion height than that of the buccal protrusions.

Furthermore, it is preferable that the oral mounting fixture has parotid gland protrusions configured to press on parts in the vicinity of the parotid glands, which are salivary glands.

Furthermore, it is desirable that the oral mounting fixture has a fitting part being fittable to at least back teeth of the dentition.

Furthermore, in the above, it is preferable that the main body section has an oral cavity antrum having an expanded shape so as to fit to the shape of a tongue or a lingual base.

Furthermore, in the above, it is preferable that the main body section and the protrusions are integrally formed by a resin.

Furthermore, in the above, it is preferable that the protrusions are coated with a metal that generates a galvanic electrical current by saliva.

### EFFECTS OF THE INVENTION

The oral mounting fixture of the present invention has plural protrusions that are integrally formed in a main body section that can be detachably mounted on a part in the oral cavity. The tips of the plural protrusions press on a mucosa in the oral cavity. Therefore, salivary glands that are broadly dispersed in the mucosa in the oral cavity are stimulated like massaging. Therefore, the secretion of saliva is further effectively promoted. Furthermore, the shape is arranged so as to conform to the shape of a portion on which the oral mounting fixture is mounted. Therefore, the oral mounting fixture can be used at any time with no limit to the timing of use. Furthermore, the oral mounting fixture can be used in public such as during conversation with other people when halitosis is of particular concern.

### BRIEF DESCRIPTION OF THE DRAWINGS

Figs. 1(A) and 1(B) are each a perspective view showing an oral mounting fixture according to the embodiment of the present invention.
Figs. 2(A), 2(B) and 2(C) are each an embodiment view showing the shape of protrusions in an embodiment.
Fig. 3 is an explanatory view explaining the positions of the salivary glands in a human face.
Figs. 4(A) and 4(B) are a perspective view and a side view showing an oral mounting fixture according to another embodiment.
Figs. 5(A), 5(B) and 5(C) are each a plane view showing an oral mounting fixture according to a still another embodiment.
Figs. 6(A), 6(B) and 6(C) are each an explanatory view that explains an example in which the salivary glands are stimulated according to an embodiment of the present invention.
Figs. 7(A) and 7(B) are each a plane view that shows an embodiment of a back teeth-fit type.
Figs. 8(A) and 8(B) are each a perspective view showing an embodiment in which the parts in the vicinity of the parotid glands are pressed on.
Figs. 9(A), 9(B) and 9(C) are each a perspective view showing an oral mounting fixture according to a still another embodiment.
Fig. 10 is an explanatory view showing a state that the oral mounting fixture is fit to a dentition.

### DESCRIPTION OF EMBODIMENTS

Saliva has effects to protect mucosae, natural purification, water content equilibration, lubrication, buffering, antibacterial, digestion, repair tissues, recalcification and prevent carcinogenesis, and the like. Saliva is essential for not only an oral cavity but also a body to exert a normal function. Furthermore, salivary glands are glands from which saliva is secreted. Ducts are opened in the oral cavity, and are classified into major salivary glands and minor salivary glands. Specifically, salivary glands include major salivary glands (parotid glands, submandibular gland and sublingual gland) and many minor salivary glands. Major salivary glands each has a tube that opens in the oral cavity, and saliva is flown out to the mouth through the tube. Minor salivary glands are widely distributed in the mucosae in the oral cavity. The outlets for saliva in the minor salivary glands are opened on the mucosae.

Furthermore, saliva is secreted from major salivary glands (parotid glands, submandibular glands and sublingual glands) and many minor salivary glands (glandulae labiales, buccales, palatinae, molar glands and linguales). The minor salivary glands include "glandulae labiales in the labial mucosae, buccales present in the buccal mucosae, anterior linguales present in the lower part of tongue tip, "posterior linguales present in the posterior parts of the lateral margins of the tongue root and tongue, as well as molar glands, and palatinae, and the like.

Saliva has a natural purification action to clean the inside of the oral cavity and an antibacterial action. Enzymes such as lysozyme suppress the propagation of bacteria to prevent generation of halitosis. Therefore, when the secretion of saliva is decreased, the inside of the oral cavity becomes dirty, and thus halitosis easily generates. The point of the present embodiment is that the secretion of less viscous saliva is maintained for a long period.

Preferable embodiments of the oral mounting fixture according to the present invention will be explained below according to the attached drawings.

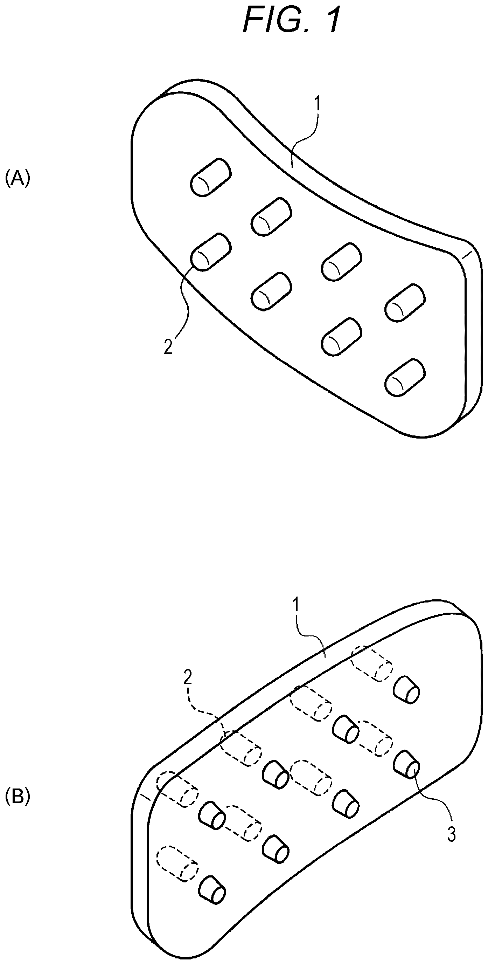

Fig. 1 is a perspective view showing an oral mounting fixture mounted in an oral cavity, which is a space in the mouth of a human, and a main body section 1, which is a thick resin, can be detachably mounted on a part in the oral cavity. The part in the oral cavity is a part "between the dentition and the lower lip", a part "between the oral cavity antrum, which is outside of the dentition, and the lower lip", specifically a part between "the dentition of back teeth and the lingual base through a gum" and "a floor of the oral cavity between the dentition and the lingual base" or the like. Fig. 1(A) is a perspective view in which the oral mounting fixture is seen from the outside in the case when the oral mounting fixture is mounted, for example, on the outside of the dentition of the like (the oral cavity part is not illustrated). Fig. 1(B) is a perspective view seen from the opposite side of Fig. 1(A) (the oral cavity part is not illustrated).

Furthermore, the shape of the main body section 1 is arranged so that any unpleasant sensation is not caused, and the main body section 1 conforms to the shape of a portion on which the oral mounting fixture is mounted. For example, as shown in Fig. 1(A), the main body section 1 slightly curves toward outside (so that the left side seen in the drawing has a convex shape). The four corners of the main body section 1 have been rounded so as to have roundness. Furthermore, the edges have been appropriately rounded at the ends in the thickness direction so that the inside of the oral cavity is not injured.

Furthermore, plural protrusions 2 and 3 have been respectively formed on the main body section 1 so that they integrally protrude from the surface. During the oral mounting fixture is mounted, the tips of the protrusions 2 and 3 press on plural positions on the mucosae in the oral cavity. By using plural protrusions 2 and 3 and allow the main body section 1 to curve, salivary glands that are widely distributed on the mucosa in the oral cavity are stimulated like massaging.

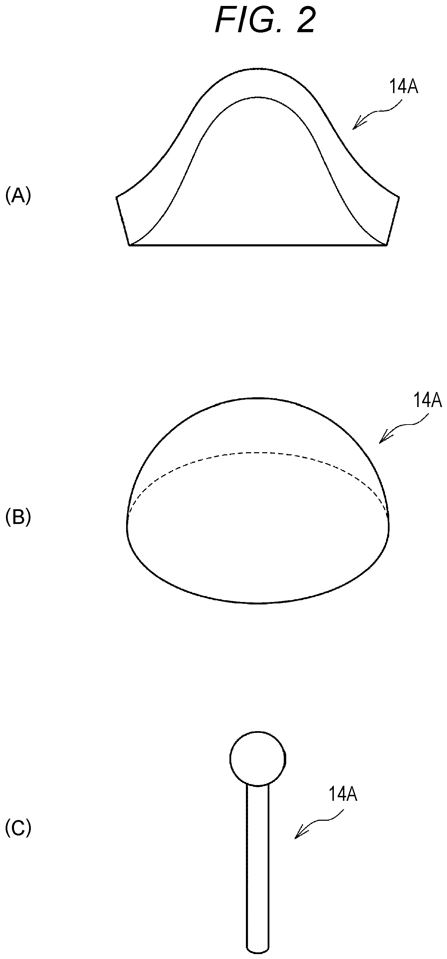

The oral mounting fixture is mounted so that the tips of the plural protrusions 2 or 3 press on at least either of an inner surface part being a buccal mucosa of the oral cavity, a submandibular part between the mandibular dentition and the lingual base and a sublingual part being the floor of the oral cavity between the dentition and the lingual base. Furthermore, the protrusions 2 each has a rod-like shape and are formed in the form of bristles from the main body section 1. The tip of the protrusion 2 presses on a mucosa on the inner surface of the oral cavity to stimulate the mucosa to thereby promote the secretion of saliva. As shown in Figs. 2(A), 2(B) and 2(C), it is preferable that the tip of the protrusion 2 is rounded so that the inner surface of the oral cavity is not injured.

Figs. 2(A), 2(B) and 2(C) each shows the shape of the tip of the protrusion 2. A semi-discoid shape as shown in Fig. 2(A), a hemispherical shape as shown in Fig. 2(B), or a shape of a rod having a tip having a spherical shape as shown in Fig. 2(C) is desirable. By imparting flexibility or elasticity to a rod-like shape or columnar shape so that it is bent, the mucosa on the inner surface of the oral cavity can be adequately massaged. Furthermore, the salivary glands are massaged more effectively by moving the mouth while the oral mounting fixture is mounted. However, the shape of the protrusion 2 of the present invention is not limited to these shapes. As the shape of the protrusion 2, various shapes such as a spherical shape and a hog-backed shape can be adopted. In summary, the protrusion may have any shape as long as the mucosa in the oral cavity can be pressed by the tip of the protrusion.

On the other hand, the protrusions 3 are minute protrusions each having a smaller protrusion height from the main body section 1 than that of the protrusions 2. The protrusions 2 are buccal protrusions that press on the buccas, which are the inner surface parts of the oral cavity. The protrusions 3 are gingiva protrusions that press on the gingivae.

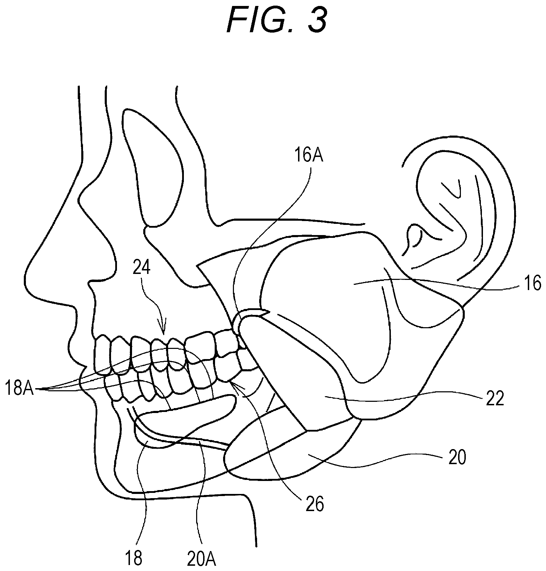

Fig. 3 is a drawing that shows the positions of the salivary glands in a human face. The salivary glands are mainly constituted by three: parotid glands 16, a sublingual gland 18 and a submandibular gland 20. The symbol 22 represents masseter muscles.

The parotid gland 16 is a salivary gland that is present by spreading in the parotid buccal part. A duct 16A that ejects saliva opens on the buccal mucosa. The sublingual gland 18 is a salivary gland that is present on the floor of the oral cavity and under the mucosa in the lingual base. Plural ducts 18A open in the vicinity of the gum. The submandibular gland 20 is a salivary gland that is present as if it partially hides in the mandibular bone. A duct 20A is opened between the mandibular dentition and the lingual base.

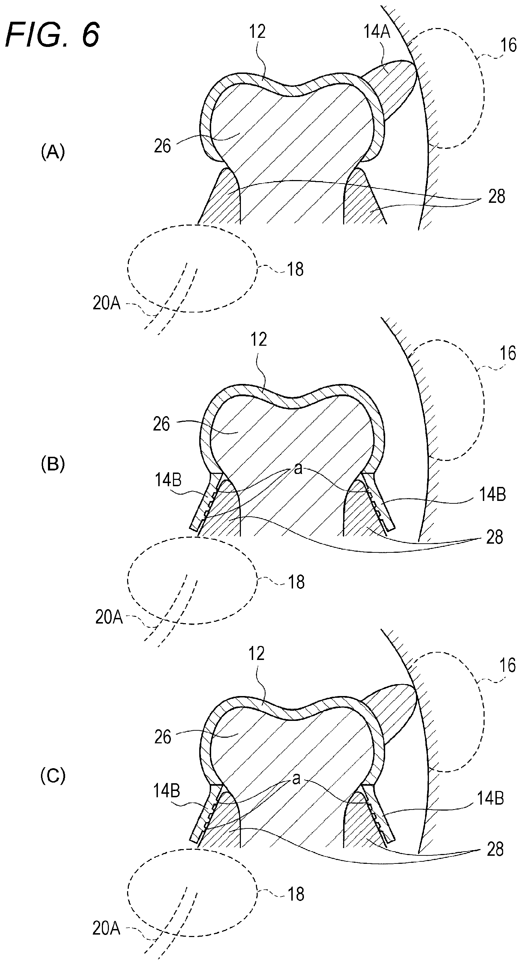

By the protrusions 3 that are minute protrusions, the part of the gum (gingiva) 28 (see Figs. 6(A), 6(B) and 6(C) for the gum), specifically the vicinities of the back teeth, is stimulated. By this way, the inner surface part of the oral cavity of the sublingual gland 18, and the duct 20A of the submandibular gland 20 can be effectively stimulated. It is preferable that the shape of the protrusion 3 is a shape such that the gum is not injured. For example, a minute protrusion having a rod-like shape having a round tip, a minute protrusion having a semicircular shape, and linear minute protrusions in which plural hog-backed bumps are arranged, and the like can be adopted. In the case of a linear minute protrusion, a shape in which plural hog-backed bumps are arranged in parallel to a dentition may also be adopted. By this way, the linear minute protrusion becomes resistance against the gum, and thus the oral mounting fixture becomes difficult to detach.

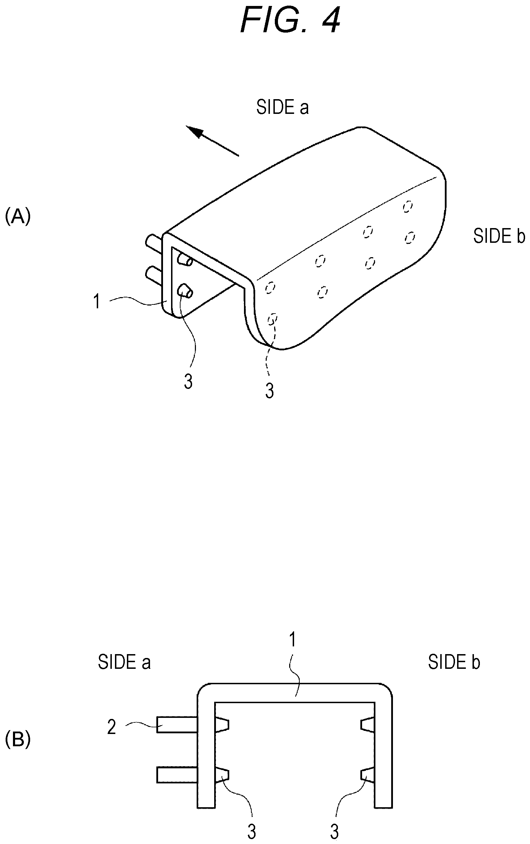

The oral mounting fixture may have a shape like a mouth piece that is tightly fitted to a dentition (for example, the shapes as shown in Figs. 4(A) and 4(B), Figs. 5(A), 5(B) and 5(C), Figs. 7(A) and 7(B), Figs. 8(A) and 8(B), and Figs. 9(A), 9(B) and 9(C)). However, it is not always necessary that the shape of the oral mounting fixture has such shape, and may have, for example, the shape as shown in Figs. 1(A) and 1(B). The oral mounting fixture is formed by, for example, a thick thermoplastic resin in the case when it is fit to a dentition, and the shape of the dentition shape may be transferred to only the inner surface (the external surface is flat). Alternatively, the oral mounting fixture may be formed of a thin thermoplastic resin so that the shape of the dentition is transferred to the entirety (the inner surface and the external surface have an identical shape). In this case, it is desirable that the oral mounting fixture is formed of a thermoplastic resin that is softened at a temperature greater than the body temperature of a human.

"Softened at a temperature greater than the body temperature of a human" means that a thermoplastic resin that forms an oral mounting fixture is softened to the extent that the shape of the dentition is transferred, when the oral mounting fixture is heated to a temperature greater than the body temperature of a human and then pressed on the maxillary dentition or mandibular dentition. Since heated water is generally preferable, the softening temperature as an actual temperature is preferably equal to or lower than 100°C, which is the boiling point of water.

Furthermore, the thermoplastic resin used may be any resin as long as safeness from a hygiene perspective is ensured and the thermoplastic resin does not have any effects such as allergy on teeth and gums.

Examples include ethylene-vinyl acetate copolymers (EVA), polyolefins (polyethylene, polypropylene, polybutadiene), polyvinyl acetates (PVA), polyurethane elastomers and the like. Among these resins, an ethylene-vinyl acetate copolymer (EVA) that satisfies a softening temperature equal to or more than 50°C, which is greater than the body temperature of a human, and an upper limit equal to or less than 100°C, which is the boiling point of temperature of water, is specifically preferable.

Figs. 4(A) and 4(B) show another embodiment, and show a perspective view (A) and a side view (B) of an oral mounting fixture having a fitting part that can be fitted to a dentition. The main body section 1 has been folded into a U-shape, and has been arranged to have a curved shape seen from above so that the lower opening is fitted to a dentition. The oral mounting fixture is mounted so that the arrow is directed to the side a, which is the outside of the dentition, i.e., the buccal mucosa, and the opposite side is directed to "the side b, which is the inner side of the dentition. Therefore, the gum part is covered with the side parts of the U-shaped convex part.

Plural protrusions 2 are formed in the form of bristles from the main body section 1 toward the outside. The tips thereof press on the inner surface part of the oral cavity, which is the buccal mucosa, in the case when the tips are fitted to a transverse side dentition. The protrusions 3 are disposed on the convex part. The protrusions 3 are minute protrusions each having a smaller protrusion height from the main body section 1 than that of the protrusions 2. The protrusions 3 press on the gum part (in the case when the main body section 1 is mounted on the mandibular dentition, the submandibular part between the mandibular dentition and the lingual base). In the case of fitting to the anterior dentition, the mounting is such that the side b is directed to the anterior direction. At this time, the protrusions 2 press on a sublingual part being the floor of the oral cavity between the dentition and the lingual base. The protrusions 3 press on the gum part.

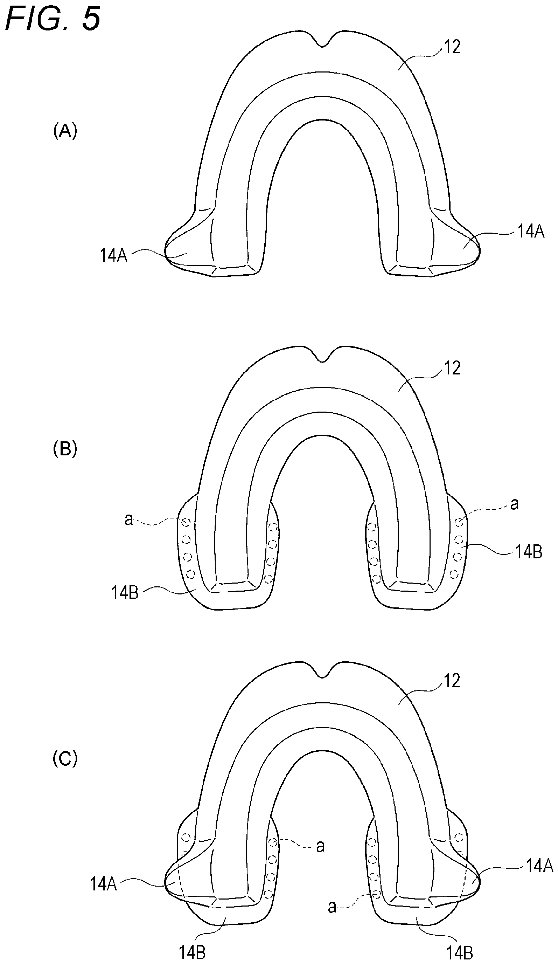

Figs. 5(A), 5(B) and 5(C) each shows an oral mounting fixture as a mouth piece 12, and this is fitted to the entirety of the mandibular dentition or maxillary dentition of a human. In the oral mounting fixture shown in Fig. 1 or 2, fitting parts are linked by a linking part, and protrusions 2 and 3 (the protrusions 2 are first projection parts 14A, and the protrusions 3 are minute protrusions a) are integrally formed. Furthermore, the mouth piece 12 of the main body section 1 is fitted to at least the back teeth parts of the maxillary dentition 24 or the mandibular dentition 26 in Fig. 3. The protrusions 14 press on at least one of the gum part in the vicinity of the back teeth and the inner surface part of the oral cavity in the vicinity of the salivary gland. By this way, the three salivary glands that secrete saliva are stimulated, whereby the secretion of saliva is promoted.

In the case when the parotid gland 16 in Fig. 3 is stimulated, the mouth piece 12 may be of a type that is fitted to either the maxillary dentition 24 or the mandibular dentition 26. However, as is understood from Fig. 3, ducts 18A and 20A from which saliva is ej ected of the sublingual gland 18 and the submandibular gland 20 are present on the dentition part of the mandible. Therefore, in order to promote the secretion of saliva at the sublingual gland 18 and the submandibular gland 20, a type that is fitted to the mandibular dentition 26 is preferable.

Figs. 5(A), 5(B) and 5(C) show preferable three embodiments wherein the protrusions 2 and 3 are projection parts. In the embodiment of the mouth piece 12 of Fig. 5(A), first projection parts 14A, which are the protrusions 2, protrude from the back teeth positions of the mouth piece 12 in the direction of the inner surface parts of parotid glands 16, which are the salivary glands of the oral cavity. Fig. 5(A) shows a drawing in which one first projection part 14A has been formed on each of the left and right back teeth positions of the mouth piece 12. However, plural first projection parts 14A may also be formed.

According to the mouth piece 12 of Fig. 5(A), as shown in Fig. 6(A), the first projection parts 14A stimulate the inner surface parts corresponding to the parotid glands 16 of the oral cavity. Therefore, the secretion of saliva from the parotid glands 16 can be promoted. In Figs. 6(A), 6(B) and 6(C), the symbol 28 represents gums.

In the mouth piece 12 of Fig. 5(B), second projection parts 14B are each a skirt-like element that is disposed to straddle the back teeth position of the mouth piece 12 so that the element covers the gum of the back teeth. Minute protrusions a that correspond to plural protrusions 3 are formed on the inner surface of the skirt-like element, which is brought into contact with the gum part of the back teeth.

According to the mouth piece 12 of Fig. 5(B), as shown in Fig. 6(B), the second projection parts 14B stimulate the inner surface part corresponding to the sublingual gland 18 of the oral cavity and the duct 20A of the submandibular gland 20, whereby the secretion of saliva from the sublingual gland 18 and the submandibular gland 20 is promoted.

In the embodiment of the mouth piece 12 of Fig. 6(C), first projection parts 14A each protrudes from the back teeth position in the direction of the inner surface parts corresponding to the parotid glands 16 of the oral cavity, and skirt-like elements each disposed to straddle the back teeth position of the mouth piece 12 to cover the gum part 28 of the back teeth, are disposed. Second projection parts 14B with minute protrusions a formed thereon have been constituted on the inner surfaces of the skirt-like elements, which are brought into contact with the back teeth.

The mouth piece 12 of Fig. 5(C) is the case when both the first projection parts 14A in Fig. 5(A) and the second projection parts 14B in Fig. 5(B) are disposed. By this way, according to the mouth piece 12 of Fig. 5(C), as shown in Fig. 6(C), three kinds of salivary glands: the parotid glands 16, the sublingual gland 18 and the submandibular gland 20 can be stimulate at the same time.

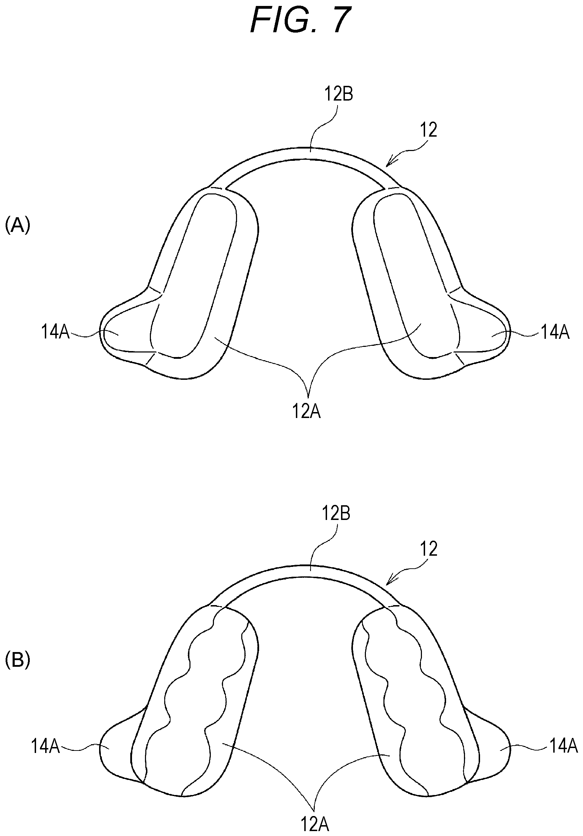

Figs. 7(A) and 7(B) show a modified example of the mouth piece 12, and is an example in which fitting parts 12A of the mouth piece 12 are disposed on only the back teeth (hereinafter referred to as "a back teeth-fit type"). Fig. 7(A) is a drawing showing the external surface of the mouth piece 12, and Fig. 7(B) is a drawing showing the inner surface. As shown in Figs. 7(A) and 7(B), the mouth piece 12 of a back teeth-fit type is constituted by a pair of fitting parts 12A and 12A, which are fitted to only the left and right back teeth, and a string-like linking part 12B having an arc-like shape, which is configured to link the fitting parts 12A and 12A.

As shown in Fig. 7(A), the external surface of the fitting part 12A is plane. As shown in Fig. 7(B), a dentition shape is formed on the inner surface of the fitting part 12A. It is preferable that the back teeth to which the fitting part 12A is fitted are three teeth: front molar, middle molar and back molar.

According to the mouth piece 12 of a back teeth-fit type, the pair of fitting parts 12A and 12A, which are fitted to only the left and right back teeth, are linked by the string-like linking part 12B. By this way, more comfortable talking is possible while the mouth piece 12 is mounted, and when the mouth is opened, the mounting of the mouth piece 12 is difficult to be seen from outside.

Protrusions 14 for promoting the secretion of saliva have been integrally formed also on the mouth piece 12 of a back teeth-fit type. In Figs. 7(A) and 7(B), the first projection parts 14A are similar to the first projection parts 14A in Fig. 5(A).

Besides the first projection parts 14A, in order to further promote the secretion of saliva, the external surfaces of the first projection parts 14A may be coated with a metal that generates a galvanic electrical current by saliva. The protrusions 2 and 3 and parotid gland protrusions 5 (mentioned below), or the entirety or a part of the main body section 1 of all of the exemplary embodiments of the present invention can be coated with the metal that generates a galvanic electrical current by saliva.

A galvanic electrical current (also referred to as a Galvani's electrical current) generally generates by the contact of different kinds of metals. However, electroconductivity has been increased by saliva in the oral cavity. Therefore, one kind of metal (for example, amalgam, gold, silver, palladium, a copper alloy, a nickel-chromium alloy, a cobalt alloy, aluminum or the like) is ionized with saliva to cause a potential difference in the oral cavity. Therefore, a galvanic electrical current, which is a faint electrical current, generates. When this galvanic electrical current generates, tingling stimulation is felt.

Accordingly, the stimulation intensity for stimulating at least one of the gum parts 28 in the vicinity of the back teeth and the inner surface parts in the vicinity of the salivary glands of the oral cavity is enhanced by coating with the metal that generates a galvanic electrical current by saliva. By this way, the secretion of saliva can further be promoted.

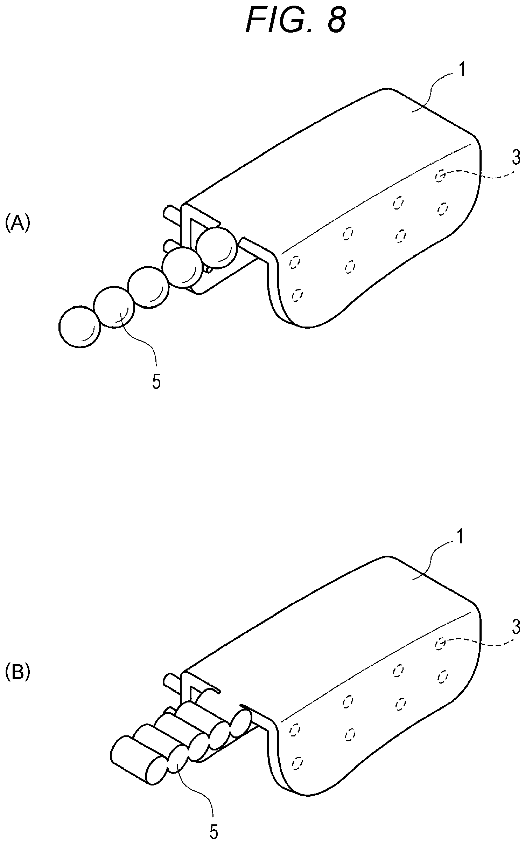

Figs. 8(A) and 8(B) show the oral mounting fixtures shown in Figs. 4(A) and 4(B), which include parotid gland protrusions 5 configured to press on parts in the vicinity of the parotid glands, which are salivary glands. In Fig. 8(A), plural spherical parts each having a spherical shape are linearly arranged in the direction of the parotid glands, which are salivary glands extending to around right in front of the ears from the main body section 1. By these spherical parotid gland protrusions 5, the parts in the vicinity of the parotid glands can be massaged by pressing.

Furthermore, this parotid gland protrusion 5 can press the gum upper surfaces (in the case of the mandible) or the gum lower surfaces (in the case of the maxilla) at the back of the middle molars or back molars (wisdom teeth). It was found by the intensive studies by the present inventors that, in the case when back molars have not teethed, the secretion of saliva is promoted by pressing on the gum upper surfaces (in the case of the mandible) or the gum lower surfaces (in the case of the maxilla) at the back of the middle molars, that is, the parts where back molars are to teethe, with the tips of the protrusions. Furthermore, they also found that, in the case when back molars have teethed, the secretion of saliva is promoted by pressing on the gum upper surfaces (in the case of the mandible) or the gum lower surfaces (in the case of the maxilla) at the back of the back molars with the tips of the protrusions. At this time, it is not always necessary that the protrusions are rod-like. As shown in the parotid gland protrusions 5 in Figs. 8(A) and 8(B), a similar effect can be obtained also by the pressing by the curved surfaces of the protrusions each having a spherical shape or a hog-backed shape.

In Fig. 8(B), the spherical parts of the parotid gland protrusion 5 are changed to hog-backed parts each having a hog-backed shape. The spherical parts in Fig. 8(A) or the hog-backed parts in Fig. 8(B) have grooves therebetween, and thus can be separated off one by one. Therefore, the length can be easily adjusted according to the size of the oral cavity of each person.

The method for using a back teeth-fit type is explained. A user immerses the oral mounting fixture in heated water retained in a heating container to soften the oral mounting fixture. In the case when the material of the oral mounting fixture is an ethylene-vinyl acetate copolymer (EVA), a temperature at which accuracy of transfer to a dentition is fine is appropriately selected in the range of heating temperature at 50 to 100°C.

Subsequently, the user presses the fitting part 12A of the oral mounting fixture in a softened state downward from the above of the left and right back teeth of the mandibular dentition 26. In this state, the user clenches the fitting part 12A together with the maxillary dentition 24. By this way, the back teeth shape is transferred to the inner surface of the main body section 1. The user then waits in this state until the oral mounting fixture is sufficiently cooled, and when the oral mounting fixture has been cooled to the body temperature, the oral mounting fixture is removed from the mandibular dentition 26. By this way, for example, the shapes of the three back teeth: front molars, middle molars and back molars are transferred in the form of grooves to the inner surface.

After the transfer, if any portion that hits to the inside of the oral cavity to cause unpleasant sensation, or the like is present, then the portion is scraped away, for example, by means of a knife or the like, whereby the shape is arranged. Thereafter, where necessary, fine adjustment by softening the oral mounting fixture again by putting it into hot water at a temperature equal to or greater than the softening point, fitting again the oral mounting fixture to the back teeth of the mandibular dentition 26 while the oral mounting fixture is soft, and, clenching the oral mounting fixture in the oral cavity. By this way, an oral mounting fixture that fits to the shape of the oral cavity of the user is formed.

Furthermore, during the main body section 1 is in a softened state, the user corrects the directions and lengths of the protrusions 2 and 3 by fingers so that the inner surface parts corresponding to the parotid glands 16 of the oral cavity are appropriately stimulated, and then solidifies the main body section 1 by natural cooling in the corrected state. Furthermore, the user corrects the directions and lengths of the protrusions 2 and 3 by fingers so that the minute protrusions a of the protrusions 3 are appropriately brought into contact with the surface of the gums 28 of the back teeth without any gap during the main body section 1 is in the softened state.

By this way, comfortable talking is possible while the oral mounting fixture is mounted on the dentitions, and the mounting of the oral mounting fixture is difficult to be seen from outside even if the mouth is opened. Furthermore, the oral mounting fixture can be used in public such as during conversation with other people when halitosis is of particular concern. Furthermore, the oral mounting fixture can be used at any time with no limit to the timing of use.

In addition, in the above-mentioned present embodiment, the user obtains a dentition shape by softening the main body section 1. It is also preferable to ask a dentist to take a more elaborate dentition shape. In the case of preparation by asking a dentist, it is also possible to use a non-thermoplastic resin material. Furthermore, a mouth piece is generally prepared through preparation of a plaster figure. It is also possible to prepare a mouth piece by means of a 3D printer as follows. Specifically, the maxillary dentition 24 and the mandibular dentition 26 are respectively photographed by a camera from at least two directions. Three-dimensional images of the maxillary dentition 24 and the mandibular dentition 26 are obtained from these photograph images. Furthermore, an oral mounting fixture can be prepared by a 3D printer by using three-dimensional coordinate data obtained from these three-dimensional images.

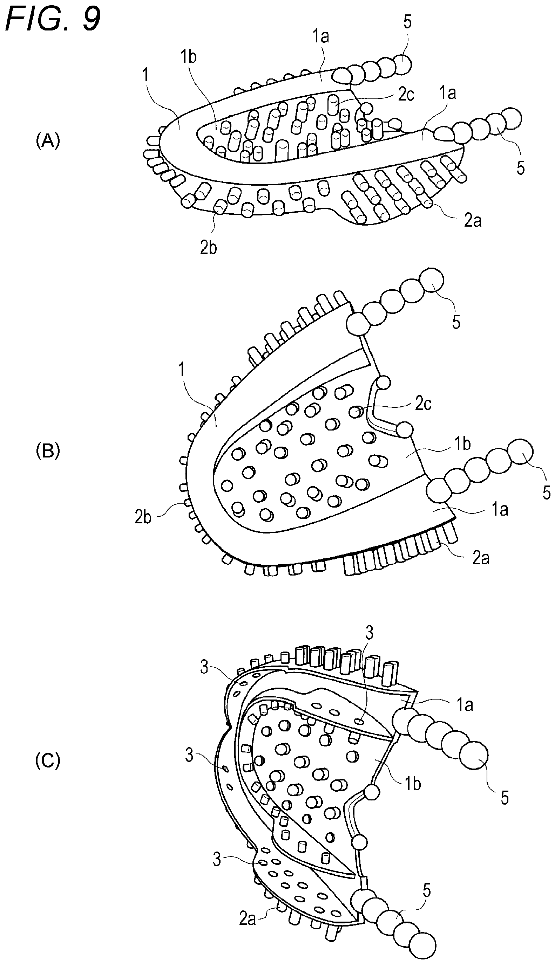

Figs. 9(A), 9(B) and 9(C) each show an example in which the oral mounting fixture is a mouth piece such that the main body section 1 is fitted to the entirety of a dentition. Fig. 9(A) is a perspective view seen from the side surface. Fig. 9(B) is a perspective view of (A) seen from another viewpoint. Fig. 9(C) is a perspective view of (A) seen from the oblique lower part. The main body section 1 is integrally formed from a back part 1a of the dentition at the left side to a back part 1a of the dentition at the right side through an oral cavity antrum 1b. The oral cavity antrum 1b is mounted so that the tongue is laid thereon, and has an expanded shape so as to conform to the shape of the tongue or lingual base. Furthermore, protrusions 2c are disposed on the upper and lower surfaces and front and rear surfaces of the oral cavity antrum 1b, and are brought into contact with the tongue or lingual base.

The size and shape of the entirety of the main body section 1 have adjusted to be a shape that gives no unpleasant sensation with the portion on which the oral mounting fixture is mounted of the user. In the back parts 1a, plural protrusions 2a that press on the buccal mucosae outward and, plural protrusions 2c that stimulate the tongue or lingual base respectively project from the main body section 1. Similarly, in the vicinity of the front teeth, plural protrusions 2b that press on the inside of the lower lip are disposed on the oral cavity antrum 1b. The protrusions 2b and protrusions 2c each has a smaller height projected from the main body section 1 than that of the protrusions 2a. By this way, each pressure for stimulation is adjusted in accordance with the movement in the oral cavity to give a suitable massage force.

Throughout the dentitions, plural protrusions 3 are disposed on the inner parts that are fitted to the gums. The protrusions 3 are minute protrusions each having a smaller protrusion height from the main body section 1 than that of the protrusions 2b. In the oral cavity antrum 1b of the main body section 1, the protrusions 2c each having a similar protrusion height to that of the protrusions 2b are disposed on the above and below. Furthermore, the projection heights may also be decreased in the order of 2a, 2b, 2c and 3.

Furthermore, parotid gland protrusions 5 configured to press parts in the vicinity of the parotid glands, which are salivary glands, are disposed. In the parotid gland protrusions 5, plural spherical parts each having a spherical shape are linearly arranged in the direction of the parotid glands, which are salivary glands extending to around right in front of the ears from the main body section 1. By the spherical parts, the parts in the vicinity of the parotid glands can be massaged by pressing. The spherical parts may also be hog-backed parts each having a hog-backed shape. Furthermore, the spherical parts or hog-backed parts have grooves so that they can separated off one by one, and thus can be adjusted according to the size of the oral cavity.

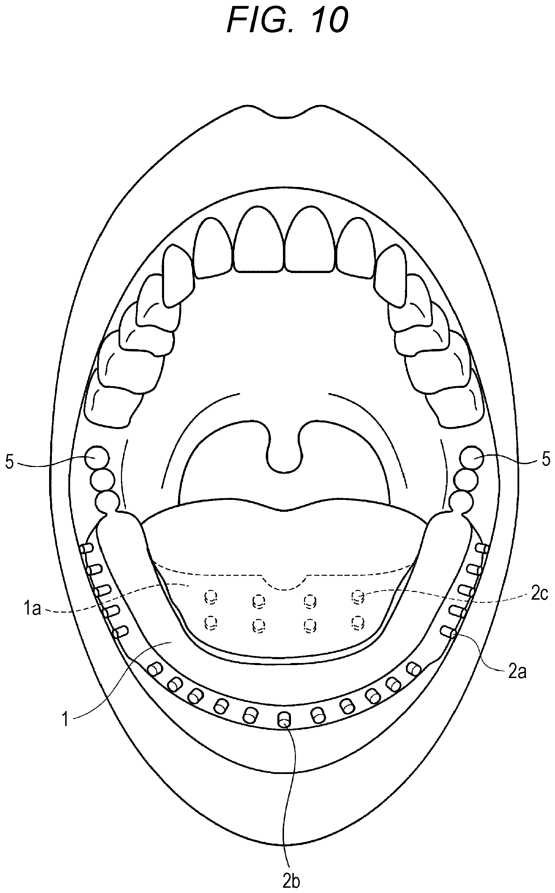

Fig. 10 shows a state in which the oral mounting fixture shown in Fig. 9 is fitted to the mandibular dentition. The main body section 1 is folded into a U-shape and fitted so that the lower opening straddles the back teeth of the mandibular dentition. The back teeth shape may be transferred by clenching the main body section 1 with the maxillary dentition 24 (see Fig. 3) in this state. The tips of the protrusions 2a directed toward the buccal sides press on the inner surface parts of the oral cavity, which are the buccal mucosae. The side of the gum is pressed by the protrusions 3 (see Fig. 9(A), 9(B) and 9(C)). The protrusions 3 are minute protrusions each having a smaller protrusion height from the main body section 1 than that of the protrusions 2. The protrusions 2b press on the inside of the lower lip around the front teeth. The tongue is laid on the oral cavity antrum 1b (see Figs. 9(A), 9(B) and 9(C)), and the protrusions 2c are brought into contact with the tongue or lingual base.

Alternatively, the oral mounting fixture of the present exemplary embodiment can be mounted on the maxillary dentition. In this case, the oral mounting fixture is mounted on the maxillary dentition in the direction of turning of the oral mounting fixture upside down from the state shown in Fig. 10. By this way, the secretion of saliva can be promoted by stimulating the upper surface of the tongue with the protrusions 2c shown in Fig. 9(A).

According to the present embodiment, not only the major salivary glands (the parotid glands, the submandibular gland and the sublingual gland) but also many glands that are widely distributed in the mucosae in the oral cavity such as the minor salivary glands, the glandulae labiales in the labial mucosa, the buccales in the buccal mucosa, the anterior linguales present in the lower part of the tongue tip, the posterior linguales present in the posterior parts of the lateral margins of the tongue root and tongue, and the molar glands and the palatinae can be appropriately stimulated. By this way, the secretion of saliva can further be effectively promoted without unpleasant sensation.

### DESCRIPTION OF REFERENCE SIGNS

1 ... main body section, 1a ... back part, 1b ... oral cavity antrum, 2 ... protrusion, 2a ... protrusion, 2b ... protrusion, 2c ... protrusion, 3 ... protrusion, 5 ... parotid gland protrusion, 12 ... mouth piece, 12A ... fitting part, 12B ... linking part, 14 ... protrusion, 14A ... first projection part, 14B ... second projection part, 16 ... parotid gland, 16A ... duct, 18 ... sublingual gland, 18A ... duct, 20 ... submandibular gland, 20A ... duct, 22 ... masseter, 24 ... maxillary dentition, 26 ... mandibular dentition, 28 ... gum (gingiva), a ... minute protrusion

## Claims

1. An oral mounting fixture configured to be mounted inside an oral cavity of a human and promote the secretion of saliva, comprising:
a main body section that can be detachably mounted on a part in the oral cavity and has a shape arranged so that the main body section conforms to the shape of a portion on which the oral mounting fixture is mounted, and
plural protrusions that are integrally formed in the main body section and protruding from the surface of the main body section,
wherein the oral mounting fixture is mounted so that the tips of the plural protrusions press on mucosae in the oral cavity.

2. The oral mounting fixture according to claim 1, which is mounted so that the tips of the plural protrusions press on at least either of inner surface parts being the buccal mucosae of the oral cavity, a submandibular part between the mandibular dentition and the lingual base, and a sublingual part being a floor of the oral cavity between the dentition and the lingual base.

3. The oral mounting fixture according to claim 1 or 2, wherein the protrusions each has a rod-like shape and are formed in the form of bristles from the main body section.

4. The oral mounting fixture according to any one of claims 1 to 3, comprising a fitting part that can be fitted to the dentition.

5. The oral mounting fixture according to any one of claims 1 to 4, wherein the main body section has plural fitting parts that can be fitted to the dentition and the fitting parts are linked by a string-like linking part.

6. The oral mounting fixture according to any one of claims 1 to 5, wherein the protrusions have plural buccal protrusions configured to press on inner surface parts of the oral cavity and plural gingiva protrusions configured to press on gingivae, wherein the gingiva protrusions each has a smaller protrusion height than that of the buccal protrusions.

7. The oral mounting fixture according to any one of claims 1 to 6, comprising parotid gland protrusions configured to press on parts in the vicinity of parotid glands, which are salivary glands.

8. The oral mounting fixture according to any one of claims 1 to 7, comprising a fitting part that can be fitted to at least back teeth of the dentition.

9. The oral mounting fixture according to any one of claims 1 to 8, comprising an oral cavity antrum having an expanded shape so as to fit to the shape of a tongue or a lingual base.

10. The oral mounting fixture according to any one of claims 1 to 9, wherein the main body section and the protrusions are integrally formed by a resin.

11. The oral mounting fixture according to any one of claims 1 to 10, wherein the protrusions are coated with a metal that generates a galvanic electrical current by saliva.
